(19) **Europäisches Patentamt / European Patent Office / Office européen des brevets**

(11) **EP 4 216 911 B1**

(12) **EUROPEAN PATENT SPECIFICATION**

(45) Date of publication and mention
of the grant of the patent:
**22.01.2025 Bulletin 2025/04**

(21) Application number: **21778056.8**

(22) Date of filing: **16.09.2021**

(51) International Patent Classification (IPC):
*A61K 8/34* (2006.01)      *A61K 8/37* (2006.01)
*A61K 8/39* (2006.01)      *A61K 8/73* (2006.01)
*A61K 8/86* (2006.01)      *A61Q 17/00* (2006.01)
*A61Q 19/00* (2006.01)

(52) Cooperative Patent Classification (CPC):
**A61Q 17/005; A61K 8/34; A61K 8/342;**
**A61K 8/345; A61K 8/37; A61K 8/39; A61K 8/735;**
**A61K 8/86; A61Q 19/00;** A61K 2800/262

(86) International application number:
**PCT/EP2021/075489**

(87) International publication number:
**WO 2022/063676 (31.03.2022 Gazette 2022/13)**

(54) **MOISTURIZING ANTIBACTERIAL COMPOSITION**

FEUCHTIGKEITSSPENDENDE ANTIBAKTERIELLE ZUSAMMENSETZUNG

COMPOSITION ANTIBACTÉRIENNE HYDRATANTE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.09.2020 IN 202021041615**

(43) Date of publication of application:
**02.08.2023 Bulletin 2023/31**

(73) Proprietors:
• **Unilever IP Holdings B.V.**
**3013 AL Rotterdam (NL)**
Designated Contracting States:
**AL AT BE BG CH CZ DK EE ES FI FR GR HR HU IS
LI LT LU LV MC MK NL NO PL PT RO SE SI SK SM**
• **Unilever Global IP Limited**
**Wirral, Merseyside CH62 4ZD (GB)**
Designated Contracting States:
**CY DE GB IE IT MT RS TR**

(72) Inventors:
• **CHANDAR, Prem**
**Trumbull, Connecticut 06611 (US)**
• **MOADDEL, Teanoosh**
**Trumbull, Connecticut 06611 (US)**
• **NEGI, Ajay, Singh**
**Bangalore 560066 Karnatak (IN)**
• **PALANISAMY, Bharath**
**Whitefield Bangalore 560 066 (IN)**
• **PATEL, Toral**
**Trumbull, Connecticut 06611 (US)**
• **SHILOACH, Anat**
**Trumbull, Connecticut 06611 (US)**
• **SINGH, Sapna**
**Mumbai 400 099 (IN)**

(74) Representative: **Fijnvandraat, Arnoldus**
**Unilever Patent Group**
**Bronland 14**
**6708 WH Wageningen (NL)**

(56) References cited:
WO-A1-2012/118055      WO-A1-2016/058902
WO-A1-2018/180009      WO-A1-2019/187703
JP-A- 2004 331 544      US-A1- 2018 193 236
US-A1- 2019 142 800

- VOLKER BÜHLER: "Pharmaceutical Technology of BASF Excipients Pharma Ingredients & Services. Welcome to more opportunities. Pharmaceutical Technology of BASF Excipients", 1 June 2008 (2008-06-01), XP055248980, Retrieved from the Internet <URL:http://www.pharma-ingredients.basf.com/documents/komplett_pharmaceutical_technology_.pdf> [retrieved on 20160210]

- SUGIURA O ET AL: "External antipruritic agent for use in formulation e.g. ointment, for treatment of e.g. dermatological disorders accompanying itching, contains allantoin, active drug substance and squalane, and/or silicone", WPI / THOMSON, 19 May 2005 (2005-05-19), XP002441004

## Description

### Field of the invention

Disclosed herein is an antibacterial composition, specifically, a transparent moisturizing antibacterial composition. The transparent moisturizing antibacterial composition includes water, alcohol, a humectant, and a sensory oil.

### Background of the invention

Consumers are increasingly concerned with microbial and viral contamination. As such, hygiene continues to be one of the most important attributes desired among consumers. Consumers desire products that will provide the required hygienic properties. Consumers throughout the world use different types of antibacterial compositions for disinfecting various surfaces including hard surfaces such as countertops, floors, and furniture; soft and porous surfaces such as clothes, carpets, and upholstery; and personal surfaces such as skin and hair. Many microorganisms like bacteria and viruses are found on such surfaces. It is desirable to keep these surfaces free of germs to minimize the risk of becoming ill.

Many antibacterial products are commercially available. Some are supplied as gels, liquids, or liquid sprays containing at least 60% by weight or more of ethanol. While such products are known to yield good antibacterial benefits after application, certain consumers shun using high ethanol-based products since such products may dry the skin, weaken the epidermis barrier, and expedite the aging process. In the current environment, consumers may be using antibacterial products on their bodies throughout the day thus increasing the likelihood of developing dry skin.

As such, there is continually a need to develop an antibacterial composition that provides excellent antimicrobial and antiviral benefits while at the same time being safe and mild enough to use an unlimited amount of times throughout the day. Given consumer demands, it is of increasing interest to develop an antibacterial composition that also delivers soothing and moisturizing benefits when topically applied. WO2016058902A1 discloses such alcoholic disinfectants that comprise a) at least 78 wt% ethanol, relative to the weight of the disinfectant, and b) one or more fatty acid esters of monovalent C1 to C6 alkyl alcohol.

### Summary of the invention

Disclosed in various aspects are moisturizing antibacterial compositions.

The invention is set out in the appended claims.

A transparent antibacterial composition comprises: water; alcohol; a humectant; and a sensory oil according to claim 1.

A method of making a transparent antibacterial composition comprises: combining a sensory oil and a humectant forming a first phase; combining water with an alcohol forming a second phase; combining the first phase and the second phase forming a third phase; and adding a neutralizer to the third phase, thereby forming the antibacterial composition according to claim 12.

These and other features and characteristics are more particularly described below.

### Detailed description of the invention

Disclosed herein is an antibacterial composition. The antibacterial composition is a transparent composition with non-tacky sensory properties. The antibacterial composition can be any form, but preferably is in the form of a transparent liquid spray. The antibacterial composition provides moisturization to the surface onto which it is applied. For example, when the composition is applied to human skin, the antibacterial composition does not leave the user with dry skin, but rather with skin that has been both moisturized and sanitized. The antibacterial composition can provide less than or equal to 8 hours of moisturization to the surface on which it is applied. The antibacterial composition can also provide antimicrobial and antiviral protection against bacteria, virus, and fungus. The antibacterial composition includes water, alcohol, a humectant, and a sensory oil. It was unexpectedly found that the antibacterial composition provides moisturization without tackiness after application. This is an unexpected benefit because antibacterial compositions typically require the use of a non-tacking agent to reduce that sticky feeling often associated with antibacterial composition application.

The antibacterial composition can be substantially free of a non-tacking agent. Substantially free of a non-tacking agent means that the composition contains less than 2% by weight of a non-tacking agent, preferably, less than 1% by weight a non-tacking agent, more preferably less than 0.5% by weight a non-tacking agent, even more preferably, less than 0.1 % by weight a non-tacking agent, still more preferably, 0% by weight a non-tacking agent. Percent by weight (% by weight) referred to herein throughout refers to the % by weight in the overall composition.

The antibacterial composition advantageously provides both moisturizing and sanitizing benefits to the surface onto which it is applied. Sanitizing or antibacterial composition as used herein refers to a composition that is capable of killing bacteria or inhibiting the growth of bacteria. The antibacterial compositions can be substantially emulsifier free. The antibacterial compositions can be transparent or translucent, preferably the antibacterial compositions are transparent. Substantially emulsifier free means that the composition contains less than 2% by weight emulsifier, preferably, less than 1% by weight emulsifier, more preferably less than 0.5% by weight emulsifier, even more preferably, less than 0.1 % by weight emulsifier, still more preferably, 0% by weight emulsifier.

The antibacterial composition comprises water in an amount of 8 to 30% by weight, preferably 10 to 25% by weight, more preferably 10 to 20% by weight.

The antibacterial composition comprises alcohol in an amount of 62 to 75% by weight, preferably 65 to 75% by weight, including all values and ranges subsumed therein. The alcohol comprises ethanol, propanol (n-propanol), isopropyl alcohol, or a combination thereof. The level of alcohol present in the antibacterial composition can be adjusted based upon the purity percentage of the alcohol. For example, if using ethanol with a purity of 92%, ethanol can be used in an amount of 70 to 75% by weight. Optionally, the alcohol can comprise chloroxylenol. A preferred alcohol can be denatured SD-40 ethanol.

The antibacterial composition can optionally comprise one or more additional antibacterial agents. The optional, additional antibacterial agent can be selected from terpenes, essential oils, or cationic oils having a solubility in water of less than 2000 parts per million (ppm) at 25°C. Examples of aromatic essential oils that can be used in the antibacterial compositions disclosed herein include amyl salicylate, carvacrol, cymene, e.g., p-cymene, dihydroeugenol, eugenol, hexyl eugenol, hexyl salicylate, isoeugenol, methyl eugenol, methyl isoeugenol, methyl salicylate, tert butyl cresol, thymol, cumarin, ylang, ylang, copaiba oil, and vanillin. Examples of non-aromatic essential oils of terpenoid compounds include cedrane, cineole, citral (including geranial and neral), citronellal, nitronelol, eucalyptol (i.e., 1,8 cineole) paradihydrolinalool, dihydromyrcenol (DH myrcenol), farnesol, geraniol, hexyl cinnamaldehyde, hydroxycitronallol, hydroxycitronellal, isocitral, limonene, preferably d-limonene, linallol, longifolene, menthol, nerol, nerolidiol, pinene, e.g., $\alpha$-pinene, phellen-drene, terpinine, e.g., $\alpha$-terpinene and $\gamma$-terpinene, terpineol, e.g., $\gamma$-terpineol and terpin-4-ol, and tetrahydromyrcenol (THM).

Preferred cationic oils include quaternary ammonium cationic vegetable oil and charged aminopolydimethylsi-lane having the formula $(CH_3)_3$-Si[Si$(CH_3)_2$-O]-[Si$(CH_3)$-$((CH_2)_3$-NH-$(CH_2)_2$-NH$_2)$-O]$_2$-Si-$(CH_3)_3$. It is preferred that the solubility of these additional antibacterial agents be less than 2000 ppm at 25°C. For example, the additional antibacterial agent can be terpineol, thymol, eugenol, borneol, limonene, or a combination thereof. A preferred additional antibacterial agent can be terpineol, thymol, or eugenol, or a combination thereof.

Other types of optional additional antibacterial agents include silver compounds. The silver compound can comprise a silver ion, for example, the silver ion can be selected from silver nitrate, silver acetate, silver oxide, silver sulfate, or a combination thereof. Preferably, the silver compound is silver nitrate.

Stated more specifically, the silver compounds optionally employed in the compositions are one or more water-soluble silver (I) compounds having a silver ion solubility of at least $1.0 \times 10^{-4}$ mol/L (in water at 25°C). Silver ion solubility, as referred to herein, is a value derived from a solubility product (Ksp) in water at 25°C, a well-known parameter that is reported in numerous sources. More particularly, silver ion solubility [Ag+], a value given in mol/L may be calculated using the formula:

$$[Ag+]=(Ksp \cdot x)^{(1/(x+1))},$$

wherein Ksp is the solubility product of the compound of interest in water at 25°C, and x represents the number of moles of silver ion per mole of compound. It has been found that silver (I) compounds having a silver ion solubility of at least $1 \times 10^{-4}$ mol/L are desirable for use herein.

Among the silver compounds desirable for use herein are silver oxide, silver nitrate, silver acetate, silver sulfate, silver benzoate, silver salicylate, silver carbonate, silver citrate and silver phosphate, or a combination thereof, preferably wherein the silver compound is silver nitrate, silver acetate, silver oxide, silver sulfate, or a combination thereof.

When present, the additional antibacterial agent can be included in an amount of 0.05 to 2% by weight, for example, 0.1 to 2% by weight of the overall antibacterial composition including all values and ranges subsumed therein.

A sensory oil can be used in the antibacterial composition to assist in leaving the user with a non-tacky feeling to the skin after application of the composition. The antibacterial composition comprises a sensory oil in an amount of 0.01 to 10% by weight, preferably 0.5 to 5% by weight including all values and ranges subsumed therein. The sensory oil can comprise a soluble sensory oil. Sensory oils desirable for use in the antibacterial composition disclosed herein must meet the following criteria: the sensory oil must be liquid, the sensory oil must be soluble in at least a 75/25 or higher ratio of EtOH/Water which is to be reflective of solubility in the final composition to give a clear final antibacterial composition, and the sensory oil must have no solvency for water.

The soluble sensory oil can comprise dicarboxylic acid esters, lactate esters, fatty alcohols, (poly)propylene glycol fatty ethers or a combination thereof. The sensory oil can also comprise mixtures of a soluble sensory oil and an insoluble sensory oil, provided the combination is soluble in the end use composition. For example, the soluble sensory oil can comprise diisopropyl adipate, diisopropyl sebacate, or diethyl hexyl malate, lauryl lactate, myristyl lactate, cetyl lactate, isostearyl alcohol, PPG14 butyl ether, PPG15 stearyl ether or a combination thereof.

Further combinations of the aforementioned with oils such as triglycerides such as capric/caprylic triglyceride or soybean oil or fatty esters such as isopropyl myristate or isopropyl palmitate are also in scope provided they are soluble and form a transparent end use composition. The latter is not limiting the choice of insoluble oil that can be used in the compositions disclosed herein. That is, other insoluble oils can be used provided that when combined with the selected sensory oils of the present compositions the insoluble oils become soluble when mixed in with the end use composition. Any combination of sensory oils used in the antibacterial compositions shall not affect the transparency of the antibacterial composition.

The antibacterial composition can additionally contain other ingredients in addition to those previously described herein including, but not limited to, skin benefit agents, fragrances, preservatives, surfactants, fixatives, opacifiers, chelators, thickening agents, humectants, structurants, dyes or colorants, or a combination thereof. For example, various colorants can optionally be used in the antibacterial composition. When present, the colorants can be in an amount of 0.00001 to 0.005% by weight of the antibacterial composition, for example, 0.0001 to 0.003% by weight, for example, 0.001% by weight of the antibacterial composition including all values and ranges subsumed therein.

For example, humectants can optionally be used in the antibacterial composition to provide additional moisturization properties to the composition. Such humectants desirable for use in the antibacterial composition can include water soluble polyols such as propylene glycol, dipropylene glycol, polypropylene glycol (e.g., PPG-9), polyethylene glycol, hydroxypropyl sorbitol, sorbitol, hexylene glycol, 1,3-butylene glycol, 1,4-butylene glycol, 1,2-octane diol, 1,2-hexane diol, isoprene glycol, 1,2,6-hexanetriol, ethoxylated glycerol, propoxylated glycerol and combinations thereof. Most preferred are glycerin, butylene glycol, propylene glycol, polyethylene glycols, sorbitol, polyglycerol, isoprene glycol, hyaluronic acid, or a combination thereof. The humectant can be present in an amount of 1 to 10% by weight, preferably 2 to 9% by weight, more preferably 3 to 8% by weight of the antibacterial composition.

The antibacterial composition can optionally contain surfactants, for example, cationic surfactants. When used, the cationic surfactant is only limited in that it should be able to be used for topical application onto human skin. The cationic surfactant can comprise branched or straight chain alkyl trimonium compounds, alkanol trimonium compounds or a combination thereof. The alkanol trimonium compounds include lauroyl ethyltrimonium methosulfate, palmitoyl ethyltrimonium methosulfate, stearoyl ethyltrimonium methosulfate, carnitine, palmitoyl carnitine, a combination thereof or the like. The trimonium compound used can be an alkyl trimonium compound comprising cetrimonium chloride, cetrimonium bromide, mytrimonium chloride, mytrimonium bromide, behentrimonium methosulfate, cocotrimonium methosulfate, behentrimonium chloride, behentrimonium bromide, steartrimonium chloride, steartrimonium bromide, laurtrimonium chloride, laurtrimonium bromide, a combination thereof or the like. For the avoidance of doubt, cationic surfactant used in the antibacterial composition disclosed herein can consist essentially of or consist of any combination of the aforementioned surfactants.

As to the cationic surfactant comprising a dimonium compound, such a compound includes dialkyl dimonium compounds like distearyl dimonium chloride, didecyl dimonium chloride, dicoco dimonium chloride, a combination thereof or the like. Other dimonium compounds suitable for use include benzethonium chloride and/or benzalkonium chloride. The dimonium and trimonium compounds used herein are meant to include salts of the same, especially chlorides and bromides of the same.

Polyquaternium materials can also be used and can include materials such as polyquaternium-6, polyquaternium-10, polyquaternium-16, polyquaternium-45, polyquaternium-28, polyquaternium-53, polyquaternium-67, acrylamidepropyl-trimonium chloride/acrylate (or acrylamide) copolymer, a combination thereof or the like.

The amount of cationic surfactant (i.e., cationic trimonium, dimonium) and/or polyquaternium material used in the composition is typically 0.007 to 5% by weight, and preferably, from 0.01 to 3% by weight, and most preferably, from 0.05 to 1% by weight, based on total weight of the composition, including all values and ranges subsumed therein.

When both trimonium and dimonium surfactant are used they are often used in a weight ratio of 1:99 to 99:1, preferably, 30:70 to 70:30, and most preferably, 40:60 to 60:40.

The antibacterial composition can also optionally include sunscreens and photostabilizers, provided that the type and amount of sunscreens and photostabilizers used do not affect the transparency of the composition. The sunscreens and photostabilizers for use include such materials as octylmethoxycinnamate (OMC), ethylhexyl salicylate, phenylbenzimidazole sulfonic acid (Ensulizole), ethylhexyl p-methoxycinnamate, available as Parsol MCX®, Avobenzene (butyl methoxydibenzoylmethane), available as Parsol 1789®, benzophenone-3, also known as oxybenzone and benzophenone-4, also known as sulisobenzone. Still others can inlcude bis-ethyl hexyloxyphenol methoxyphenol triazine, 2-ethylhexyl-2-cyano3,3-diphenyl-2-propanoic acid, drometrizole trisiloxane, 3,3,5-trimethyl cyclohexyl 2-hydroxybenzoate, 2-ethylhexyl-2-hydroxybenzoate or combination thereof. Inorganic sunscreen actives may be employed such as microfine titanium dioxide (preferably with a particle diameter of less than 150 nanometers (nm), and most preferably, less than 100nm) and zinc oxide may be used, polyethylene and various other polymers are also suitable sunscreens. Other sunscreens suitable for use include p-aminobenzoic acid (PABA), octyldimethyl-PABA, 2-ethoxyethyl p-methoxy cinnamate, benzophenone-1, benzophenone-2, benzophenone-6, benzophenone-8, benzophenone-9, benzophenone-12, homomethyl salicylate, menthyl anthranilate, benzophenone-4, triethanolamine salicylate, terephthalylidene dicamphor sulfonic acid, bisoctriazole, bisethylhexyloxyphenol methoxyphenyl triazine, bisdisulizole disodium, diometriazole trisiloxane, octyltriazone, iscotrizinol, polysilicone-15, isopentenyl-4-methoxycinnamate, or a combination thereof. Octocrylene can also be used. Amounts of the sunscreen or photostabilizing agents when present can be 0.001 to 20%, preferably, 0.005 to 15%, more preferably 0.01 to 0.2%, even more preferably, 0.1% by weight of the antibacterial composition including all values and ranges subsumed therein.

Desirably the optional skin benefit agents used in the antibacterial composition disclosed herein include niacinamide (vitamin $B_3$), tocopherol (Vitamin E), aloe vera, alpha-hydroxy acids and esters, beta-hydroxy acids and esters, hydroxyethyl urea, polyhydroxy acids and esters, creatine, hydroquinone, t-butyl hydroquinone, mulberry, hyaluronic acid and salts thereof (including, but not limited to, Na+ and K+ salts of the same), extract, liquorice extract, resorcinol derivatives, or a combination thereof. Such skin benefits can include, but are not limited to, wound healing benefits like healing, dryness reduction, anti-sallowing, etc. For example, the skin benefit agent can be sodium hyaluronate. Such benefit agents, including sodium hyaluronate can be present in an amount of 0.0001 to 10%, for example, 0.001 to 6.5%, for example, 0.01 to 3.5%, and for example, 0.01% by weight, based on total weight of the antibacterial composition and including all values and ranges subsumed therein.

Further optional water-soluble skin benefit agents include acids, such as amino acids like arginine, valine, histidine, lysine, aspartic acid, threonine, serine, glutamic acid, proline, glycine, alanine, isoleucine, leucine, tyrosine, or phenylalanine. Other vitamins can be used such as vitamin $B_2$, picolinamide, panthenol (vitamin $B_5$), vitamin $B_6$, vitamin C, a combination thereof or the like. Derivatives (generally meaning something that has developed or been obtained from something else), and especially, water soluble derivatives of such vitamins can also be employed. For instance, vitamin C derivatives such as ascorbyl tetraisopalmitate, magnesium ascorbyl phosphate and ascorbyl glycoside may be used alone or in combination with each other. Niacinamide derivatives such as nicotinamide adenine dinucleotide (NADH) and nicotinamide adenine dinucleotide phosphate (NADPH) may be used alone or in combination with each other. Other skin benefit agents that can be used include 4-ethyl resorcinol, extracts like sage, aloe vera, green tea, sugar cane, citrus, grapeseed, thyme, chamomile, yarrow, cucumber, liquorice, rosemary extract, or a combination thereof. Electrolytes such as NaCl and/or KCl, $MgCl_2$ may also be used. The total amount of optional water-soluble benefit agents (including mixtures) when present in the composition disclosed herein can be 0.0001 to 10%, preferably, 0.001 to 6.5%, and most preferably, 0.01 to 3.5% by weight, based on total weight of the antibacterial composition and including all values and ranges subsumed therein.

It is also within the scope of the antibacterial composition to optionally include oil soluble benefit agents. Illustrative examples of the types of oil soluble benefit agents that can optionally be used in the antibacterial composition disclosed herein include components like stearic acid, vitamins like vitamin A, D, E and K (and their oil soluble derivatives).

Other optional oil soluble benefit agents for use include resorcinols and resorcinol derivatives like 4-hexyl resorcinol, 4-phenylethyl resorcinol, 4-cyclopentyl resorcinol, 4-cyclohexyl resorcinol 4-isopropyl resorcinol or a combination thereof. Also, 5-substituted resorcinols like 4-cyclohexyl-5-methylbenzene-1,3-diol, 4-isopropyl-5-methylbenzene-1,3-diol, combination thereof or the like may be used. The 5-substituted resorcinols and their synthesis are described in commonly assigned U.S. Published Patent Application No. 2016/0000669A1.

Even other oil soluble benefit agents that can be used include omega-3 fatty acids, omega-6 fatty acids, climbazole, magnolol, honokiol, farnesol, ursolic acid, myristic acid, geranyl geraniol, oleyl betaine, cocoyl hydroxyethyl imidazoline, hexanoyl sphingosine, 12-hydroxystearic acid, petroselinic acid, conjugated linoleic acid, stearic acid, palmitic acid, lauric acid, terpineol, thymol essential components, the dissolution auxiliary selected from limonene, pinene, camphene, cymene, citronellol, citronellal, geraniol, nerol, linalool, rhodinol, borneol, isoborneol, menthone, camphor, safrole, isosafrole, eugenol, isoeugenol, tea tree oil, eucalyptus oil, peppermint oil, neem oil, lemon grass oil, orange oil, bergamot oil, or a combination thereof.

Another optional oil soluble benefit agent that may be used is a retinoic acid precursor. The retinoic acid precursor can be retinol, retinal, retinyl ester, retinyl propionate, retinyl palmitate, retinyl acetate or a combination thereof. Retinyl propionate, retinyl palmitate and combinations thereof are typically preferred. Still another retinoic acid precursor for use is hydroxyanasatil retinoate made commercially available under the name Retextra® as supplied by Molecular Design International. The same may be used in a combination with any of the oil soluble benefit agents described herein.

When an optional (i.e., 0.0 to 1.5% by weight) oil soluble benefit agent is used in the antibacterial composition, it typically is present in an amount of 0.001 to 1.5%, and for example, 0.05 to 1.2%, for example, 0.2 to 0.5% by weight of the total weight of the end use composition, including all values and ranges subsumed therein.

Film forming agents may be used in the antibacterial compositions. While optional, such agents can aid with the composition adhering to the surface to which it is applied. Film forming agents include those having hydrophilic properties and they include materials comprising polyvinylpyrrolidone (PVP), acrylates, acrylamides, and copolymers thereof.

Deposition agents like organosiloxanes and polyquaternium-7 (Merquat™ S Polymer from Lubrizol) may also be used. When used, such agents make up from 0.001 to 1% by weight of the antibacterial composition including all values and ranges subsumed therein.

Other optional components that can be used in the composition are anti-mosquito agents like eucalyptus oil, lavender oil, N,N-diethyl-meta-toluamide (DEET), a combination thereof or the like. Even other ingredients which may be used include octopirox (piroctone), zinc pyrithione, chloroxylenol, triclosan, cetylpyridinium chloride as well as silver compounds including silver oxide, nitrate, sulfate, phosphate, carbonate, acetate, benzoate, a combination thereof or the like. If used, these other components typically make up from 0.001 to 1.6%, and preferably, from 0.01 to 1.2% by weight of the antibacterial composition including all values and ranges subsumed therein.

Optionally, preservatives can be used in the antibacterial composition disclosed herein. When used, illustrative preservatives for use include sodium benzoate, iodopropynyl butyl carbamate, phenoxyethanol, hydroxyacetophenone, ethylhexylglycerine, methyl paraben, propyl paraben, imidazolidinyl urea, sodium dehydroacetate, dimethyl-dimethyl (DMDM) hydantoin and benzyl alcohol or a combination thereof. Other preservatives suitable for use include sodium dehydroacetate, chlorophenesin and decylene glycol. Preservatives are preferably employed in amounts of 0.01% to 2.0% by weight of the total weight of antibacterial composition, including all values and ranges subsumed therein. Also preferred is a preservative system with hydroxyacetophenone alone or in a mixture with other preservatives.

Fragrances, fixatives, opacifiers (like titanium dioxide or glycol distearate), chelating agents may optionally be included in the antibacterial composition. Possible chelating agents include, but are not limited to, ethylyene diaminetetraacetic acid (EDTA), diethylenetriaminepentaacetic acid (DTPA), ethylene diamine disuccinic acid (EDDS), pentasodium diethylenetriaminepentaacetate, trisodium N-(hydroxyethyl)-ethylenediaminetracetate, an acid form of EDTA, sodium thiocynate, trisodium salt of methylglycinediacetic acid, tetrasodium glutamate diacetate and phytic acid, preferably wherein the chelating agent is ethylene diaminetetraacetic acid (EDTA), diethylenetriaminepentaacetic acid (DTPA), ethylene diamine disuccinic acid (EDDS), or a combination thereof. Each of these substances may be present in an amount of about 0.03 to about 3%, preferably, about 0.1 to about 2.6% by weight of the total weight of the antibacterial composition, including all values and ranges subsumed therein.

Another optional additive desirable for use includes hemp oil with 2.5 to 25% by weight cannabigerol and/or cannabidiol at from 0.5 to 10 percent by weight. When used, such oil makes up 0.0001 to 1.5% by weight of the antibacterial composition, and preferably, 0.01 to 1% by weight of the antibacterial composition, if used, including all values and ranges subsumed therein.

To adjust antibacterial spray composition viscosity, it is within the scope of the compositions to optionally include small amounts of thickening agents. Optional for use are thickeners classified as polysaccharides. Examples include fibers, starches, natural/synthetic gums and cellulosics. Representative of the starches are chemically modified starches such as sodium hydroxypropyl starch phosphate and aluminum starch octenylsuccinate. Tapioca starch is often preferred, as is maltodextrin. Gums include xanthan, tara, sclerotium, pectin, karaya, arabic, agar, guar (including Acacia senegal guar), carrageenan, alginate and combinations thereof. Cellulosics include hydroxypropyl cellulose, hydroxypropyl methylcellulose (e.g., BENECEL™ E10M by Ashland), ethylcellulose, sodium carboxy methylcellulose (cellulose gum/carboxymethyl cellulose) and cellulose (e.g., cellulose microfibrils, cellulose nanocrystals or microcrystalline cellulose). Sources of cellulose microfibrils include secondary cell wall materials (e.g., wood pulp, cotton), bacterial cellulose, and primary cell wall materials. Preferably the source of primary cell wall material is selected from parenchymal tissue from fruits, roots, bulbs, tubers, seeds, leaves and combination thereof; more preferably is selected from citrus fruit, tomato fruit, peach fruit, pumpkin fruit, kiwi fruit, apple fruit, mango fruit, sugar beet, beet root, turnip, parsnip, maize, oat, wheat, peas and combinations thereof; and even more preferably is selected from citrus fruit, tomato fruit and combinations thereof. A most preferred source of primary cell wall material is parenchymal tissue from citrus fruit. Citrus fibers, such as those made available by Herbacel® as AQ Plus can also be used as source for cellulose microfibrils. The cellulose sources can be surface modified by any of the known methods including those described in Colloidal Polymer Science, Kalia et al., "Nanofibrillated cellulose: surface modification and potential applications" (2014), Vol 292, Pages 5-31.

Other thickening agents that can be included can comprise a hydrophobically modified crosspolymer. Synthetic polymers are effective thickening agents. Possible thickening agents includes crosslinked polyacrylates such as the carbomers like ASHLAND™ 980 carbomers (cross linked polymer of acrylic acid), acrylate copolymers, acrylates/ acrylate ($C_{10}$-$C_{30}$) alkyl acrylate crosspolymers such as the Carbopol® line like Ultrez20 and ETD2020 commercially available from Lubrizol with an INCI name of acrylates/C10-30 alkyl acrylate crosspolymer. Still other polymers from Lubrizol Carbopol® line can include Ultrez 10, Ultrez 21 and the like. Polyacrylamides such as Sepigel® 305 and taurate copolymers such as Simulgel® EG and Aristoflex® AVC, the copolymers being identified by respective INCI nomenclature as sodium acrylate/sodium acryloyldimethyl taurate and acryloyl dimethyltaurate/vinyl pyrrolidone copolymer. Still other thickening polymers can include synthetic polymers such as an acrylate-based polymer made commercially available by Seppic and sold under the name Simulgel INS100. Calcium carbonate, fumed silica, and magnesium-aluminum-silicate may also be used.

The amounts of the thickening agent, when used, may range from 0.001 to 5%, by weight of the antibacterial composition. Maltodextrin, xanthan gum, and carboxymethyl cellulose are the often preferred thickening agents. Other thickening agents often desired are dilinoleyl/dimethyl carbonate copolymer, stearyl alkonium hectorite, cetyl (cetearyl, stearyl) fatty alcohols, tara gum, polyquaternium 32 and/or 37, pentaerythrityl tetrastearate or a combination thereof.

The antibacterial composition can optionally contain an emulsifier. The emulsifier may be selected from the group consisting of those with a $C_{10}$-$C_{20}$ fatty alcohol or acid hydrophobe condensed with about 2 to about 100 moles of ethylene oxide or propylene oxide per mole of hydrophobe; $C_2$-$C_{10}$ alkyl phenols condensed with 2 to 20 moles of alkylene oxide; mono- and di-fatty acid esters of ethylene glycol; f; sorbitan, mono- and di- $C_8$-$C_{20}$ fatty acids; and polyoxyethylene sorbitan, or a combination thereof. Alkyl polyglycosides and saccharide fatty amides (e.g., methyl gluconamides) can also be used as nonionic emulsifiers.

Preferred emulsifiers typically have an HLB (hydrophilic-lipophilic balance) of 7.5 to 28, and preferably, 8 to 25, and most preferably, 9 to 20, including all ranges subsumed therein. e.g., nonionic emulsifier can include polysorbate 20 (Tween 20), polyoxyethylene (20) sorbitan monooleate (Tween 80). When present, the emulsifier can be present in an amount of 0 to 1% by weight, for example, 1% by weight including all values and ranges subsumed therein.

The antibacterial composition can additionally contain a neutralizing agent such as sodium hydroxide, potassium hydroxide, triethanolamine, ammonium, arginine, tromethamine, aminomethyl propanol sold as AMP 95, AMP-Ultra PC1000, AMP-Ultra PC2000, AMP-Ultra PC3000 by Angus, tetrahydroxylpropyl ethylenediamine also known as Neutrol TE from BASF, diisopropanolamine, triisopropanolamine, or a combination thereof. The neutralizing agent can be present in an amount of 0 to 1% by weight, for example, 0.1 to 0.75% by weight, for example, 0.2 to 0.5% by weight including all values and ranges subsumed therein.

As to packaging, the antibacterial composition, can be packaged in a spray bottle, squeeze bottle, or provided as an impregnating wetting agent on cotton swab, wipe, towelette, cosmetic substrate sheet (like those described in US 6,294,182 B1) or the like. As the viscosity is increased with thickening agent, the antibacterial composition gels and may be provided to consumers in a squeeze bottle as a gel composition. The spray bottle may also be metal, and the antibacterial composition may be provided via conventional aerosol packaging technologies and including those which utilize air-in-bag discharging cannisters, mechanisms and actuators. It is also within the scope of the antibacterial composition to include foaming agents (e.g., zwitterionic and/or amphoteric surfactants) so that the antibacterial composition can be discharged as a foam.

The antibacterial composition should be supplied with instructions to apply (e.g., squeeze or spray) the composition on to a surface, like skin, for bacteria kill and viral activity reduction. The antibacterial composition can be provided for in biodegradable packaging and the packaging used is preferably refillable or reusable, biodegradable, and/or at least 50%, and preferably, at least 100% made from post-consumer recycled resin. The antibacterial composition can be in the form of a liquid or a gel, preferably the antibacterial composition is in the form of a liquid, e.g., a liquid spray antibacterial composition.

Skin, as used herein, is meant to include skin on the arms (including underarms), face, feet, neck, chest, hands, legs, buttocks and scalp (including hair). Sanitizing as used herein means a bacteria log kill of at least 2 and a viral log inactivation of at least 2 (both achieved), preferably a bacteria log kill of at least 3 and a viral log inactivation of at least in less than 3 minutes after topical application to a surface. Combination, as used herein means, total weight, e.g., of cetrimonium chloride plus benzalkonium chloride. Skin benefit agent means an ingredient suitable to improve a skin characteristic. Surface as used herein includes skin or the surface of an inanimate object such as a tabletop, computer monitor, doorknob, toilet seat, shopping cart handle or even a clothing garment. Surface is also meant to include the coat of an animal such as the fur on a dog and cat. As used herein, surface preferably means human skin, and especially, skin on the face and hands.

The antibacterial composition can be a home care composition like a laundry spray composition suitable to spray clothing and upholstery requiring sanitizing. The home care composition can also be an antibacterial kitchen or bathroom spray composition. Preferably, the antibacterial composition is a topical composition to apply to skin for sanitizing by significantly reducing the amount of bacteria and viruses on the skin. The composition may optionally comprise skin benefit ingredients added thereto such as emollients, vitamins and/or derivatives thereof, resorcinols, retinoic acid precursors, colorants, moisturizers or humectants, fragrances, sunscreens, a combination thereof or the like as previously described herein. The skin benefit ingredients may be water or oil soluble.

The antibacterial composition, therefore, is a hydroalcoholic based composition with a pH of 3.0 to 8.2, and the composition is water/alcohol continuous. Viscosity, as used herein, is taken either with a Brookfield viscometer using Spindle 4 at 10 rpm or with a Discovery HR-2 Rheometer using sand blasted plates having a 1000 micron gap and a first shear rate $S_A$ of 0.4 s$^{-1}$ for a first viscosity $V_A$ and a second shear rate $S_B$ of 10 s$^{-1}$ for a second viscosity $V_B$, both at 25°C and 20 second interval.

Several units are known to be used in the context of viscosity, but the ones used more often are centipoise (cP), Pascal seconds (Pa*s) and millipascal seconds (mPa*s) and these units are easily interconvertible with the help of publicly available resources like textbooks, encyclopaedias and the internet. In still another embodiment, the composition is a non-therapeutic and non-medicinal composition which is a hydroalcoholic solution having a viscosity under 10,000 mPa*s (cP) (10 Pa*s), preferably having a viscosity under 5,000 mPa*s (5 Pa*s), preferably having a viscosity of 0.8 to 3,000 mPa*s (0.0008 to 3 Pa*s), more preferably having a viscosity of 0.8 to 2,000 mPa*s (0.0008 to 2 Pa*s).

Typically, the viscosity of the antibacterial composition will be under 10,000 mPa*s (10 Pa*s).

Often the viscosity of the antibacterial composition will be 0.8 to 5,000 mPa*s (0.0008 to 5 Pa*s), and preferably, 0.8 to 3,000 mPa*s (0.0008 to 3 Pa*s), more preferably, 0.8 to 2,000 mPa*s (0.0008 to 2 Pa*s), and still more preferably, 0.8 to 1,000 mPa*s (0.0008 to 1 Pa*s), including all ranges subsumed therein.

The antibacterial compositions can be made by any method of making an antibacterial composition. In one embodiment, a method of making the antibacterial composition can include the following: combining the sensory oil and a humectant to form a first phase.

Water is then combined with the alcohol to form a second phase. The first and second phases are then combined to form a third phase. A neutralizer is added to the third phase, thereby forming the antibacterial composition.

## Examples

The following examples are merely illustrative of the antibacterial compositions disclosed herein and are not intended to limit the scope hereof.

Example 1:

Samples 1 and 2 are examples demonstrating compositions of the antibacterial compositions disclosed herein.

Table 1 - All numbers are shown as weight %

| | Sample 1 | Sample 2 |
|---|---|---|
| Ingredient | | |
| Ethyl Alcohol I.P. SDA 40B, 190 proof denatured with denatonium benzoate (Bitrix) and tertiary butyl alcohol | 72 | 72 |
| Niacinamide I.P. | 0.2 | 0.2 |
| Vitamin E Acetate I.P. | 0.05 | 0.05 |
| Tetrasodium EDTA | 0.005 | 0.005 |
| Glycerine | 5 | 5 |
| Diisopropyl adipate | 3 | 5 |
| 1,3 Butylene glycol | 3 | 3 |
| Aloe vera extract | 0.1 | 0.1 |
| Color | 0.001 | 0.001 |
| Benzophenone-4 | 0.1 | 0.1 |
| Sodium hyaluronate | 0.001 | 0.001 |
| Perfume | 0.7 | 0.7 |
| Water | Q.S. | Q.S. |

The level of alcohol present in the antibacterial composition can be adjusted based upon the purity percentage of the alcohol. For example, the ethanol purity should preferably be 92% w/w to 94% w/w such that if using ethanol with a purity of 92%, the ethanol can be used in an amount of 70 to 80% by weight, for example, 72% by weight.

Table 2 shows oils that can be used in the antibacterial compositions to design a clear and quick drying hand sanitizer spray formulation with a non-tacky feel. The examples are not meant to limit the materials that can be used but merely to illustrate oil type and level selected based on three criteria. The oils that are preferred for use are oils that have greater than 30% solubility in at least a 75/25 EtOH/Water blend reflective of good solubility in formulation shown in Sample 1. Furthermore, the oil must be liquid at room temperature so that once left behind as a film, the composition does not impart any negative feel. Finally, the preferred oil must itself not have solvency for water such that during application and on drying as the ethanol and water evaporate, the water is also allowed to quickly evaporate leaving behind only the oil and any skin benefit actives/moisturizers if present. Without wishing to be bound by theory it is believed that when a hand sanitizer formulation with preferred oils as disclosed herein is applied to the skin and as the water and ethanol evaporate, if the oil possesses good solubilizing capacity for water, this will delay evaporation and drive towards an undesirable slower drying and tackier feel.

Table 2

| Sensory oil | Solubility in 75/25 EtOH/Water | Water solubility in sensory oil | Oil (liquid or solid at RT) | In or out of scope |
|---|---|---|---|---|
| Dicapryl maleate | <10% | No | Liquid | Out |
| Dioctyl succinate | <10% | No | Liquid | Out |
| Diethyl hexyl sebacate | <10% | No | Liquid | Out |
| Diethylhexyl adipate | <10% | No | Liquid | Out |
| PPG-2-Isoceteth-2 | >30% | Yes | Liquid | Out |
| PPG15 Stearyl ether | >30% | No | Liquid | In |
| Glycereth-5 lactate | >30% | Yes | Liquid | Out |
| Glycereth-7 lactate | >30% | Yes | Liquid | Out |
| Disopropyl adipate | >30% | No | Liquid | In |
| Diisopropyl sebacate | >30% | No | Liquid | In |
| Lauryl lactate | >30% | No | Liquid | In |
| Lauryl lactate/CCT (80/20) | <10% | No | Liquid | Out |
| Lauryl lactate/CCT (95/5) | >30% | No | Liquid | In |
| CCT | <10% | No | Liquid | In |
| Lauryl lactate / IPM (80/20) | >30% | No | Liquid | In |
| Lauryl lactate / IPM ( 5/50) | <10% | No | Liquid | Out |
| Diethyl hexyl malate / IPM (50/50) | <10% | No | Liquid | Out |
| Isostearyl alcohol | >30% | No | Liquid | Out |
| Isocetyl alcohol | <10% | No | Liquid | Out |
| Diisopropyl adipate / IPM 50/50 | <10% | No | Liquid | Out |
| Diethyl hexyl malate | >30% | No | Liquid | In |
| Glyceryl Monoleate | >30% | yes | Solid | Out |
| PPG-2 Isoceteth-20 Acetate | >30% | no | Solid | Out |

Demonstrated in Table 2 are examples of oils that satisfy and do not satisfy the three criteria, which include: (1) oil must be liquid; (2) oil must be soluble in at least a 75/25

EtOH/Water mixture; (3) oil must have no solvency for water and thus, are desirable for use in the disclosed antibacterial compositions.

Example 2:

The composition of Sample 2 was used for testing of moisturization, resilience wound healing testing, and melanin content.

Moisturization was measured with visual measurements (dryness and erythema) and instrumental hydration assessments with a Skicon and Corneometer. Measurements were obtained at a baseline, 2 hours, 4 hours, 6 hours, and 8 hours after a single controlled product application.

As can be seen from the data, the sanitizer made with the composition of Sample 2 showed improvements in hydration compared to the baseline at all time points. The sanitizer made with the composition of Sample 2 was also superior in hydration compared to the untreated control sites.

Sample 2 was tested and compared to Samples 3 and 4. Formulations for Samples 2 to 4 are displayed in Table 3. Samples 4 and 5 were untreated skin to which no sanitizing composition was applied.

Table 3

| | Sample 2 | Sample 3 | Sample 4 |
|---|---|---|---|
| Ingredient | | (untreated) | (untreated) |
| Ethyl Alcohol I.P. SDA 40B, 190 proof denatured with denatonium benzoate (Bitrix) and tertiary butyl alcohol | 72 | - | - |
| Niacinamide I.P. | 0.2 | - | - |
| Vitamin E Acetate I.P. | 0.05 | - | - |
| Tetrasodium EDTA | 0.005 | - | - |
| Glycerine | 5 | - | - |
| Diisopropyl adipate | 5 | - | - |
| 1,3 Butylene glycol | 3 | - | - |
| Aloe vera extract | 0.1 | - | - |

| | Sample 2 | Sample 3 | Sample 4 |
|---|---|---|---|
| Color | 0.001 | - | - |
| Benzophenone-4 | 0.1 | - | - |
| Sodium hyaluronate | 0.001 | - | - |
| Perfume | 0.7 | - | - |
| Water | Q.S. | - | - |

Participants were entered into a 5-day conditioning phase where they were instructed to use DOVE® white soap bar for all general bathing and showering including the test sites (forearms). Participants were asked to refrain from using any additional products on the test areas during the study and from shaving within two days prior to or during the product application and assessment phase. Participants were also instructed to show at least 12 hours prior to a study.

At the baseline visit, participants with visual dryness scores of 0 to 2.5 and erythema scores ≤2.0 (0-6 scale) on each site on the volar forearms with a difference in grades not exceeding ± 0.5 between adjacent sites or 1.0 unit overall were enrolled in the product application phase. A total of eight test sites (4 cm × 4 cm) were randomly assigned to either the right or left volar forearm within a participant. The treatment sites were balanced across all sites on the volar forearm via a carry-over latin square design.

Prior to the product application phase, baseline assessments were obtained for Visual assessment of dryness and erythema by an expert visual assessor and instrumental assessment of hydration (Skicon: conductance, Corneometer: capacitance). Following baseline measurements, study personnel made a single controlled product application. The product application procedure is described as follows:

Study personnel dispensed 0.035 mL of the test product to the test site using a repeating pipette. Using gloved fingers, the test product was thoroughly rubbed onto the assigned test site in a circular motion until the site was completely wet. The site was then allowed to air dry.

Two test sites were left untreated on each subject, which form Samples 3 and 4.

Visual assessment of dryness, Corneometer, and Skicon measurements were then obtained at 2, 4, 6 hours (± 15 minutes), and 8 hours (± 30 minutes) post application. Participants acclimated for a minimum of 20 minutes prior to assessments in a room maintained at 66.3°F to 70.1°F (19.0°C to 21.2°C) and 38% to 55% relative humidity. One trained evaluator performed all visual evaluations during the treatment phase. More than one trained operator per instrument was used to collect instrument readings.

Visual dryness analysis was completed using simple pairwise non-parametric methods (Wilcoxon Signed-Rank test with Pratt-Lehman adjustment), for example assessing within-product Change from Baseline (CFB) or assessing the difference in CFB for pairs of products.

For instrumental data, within each product/untreated site, each evaluation timepoint was compared to baseline using a paired t-test, 2-tailed, at 95% confidence level. Between-treatment analyses for instrumental measurements applied a single linear model at each timepoint (with CFB as the outcome variable, BL as a covariate and PRODUCT as the effect of interest) comparing all products to all others. CFB for each product, and also all product comparisons were conducted for all comparisons of interest.

No visual erythema was observed during the study for any of the Samples or untreated site.

During hydration assessment with the Corneometer, Samples 2 to 4 were evaluated. As seen from Table 4, Samples 3 and 4 showed either no change from the baseline or worsening Corneometer levels at all timepoints after application. Sample 2 showed improvement at all timepoints after one application.

Table 4: Corneometer CM825 Skin Hydration CFB Values at Volar Forearm

| Sample # | Time (4hr) | Mean CFB | Est. | p-value (two sided) | % Subjects improvements from BL | Mean % improvement from BL | Conclusion |
|---|---|---|---|---|---|---|---|
| 2 | 2 | 5.06 | 5.06 | <0.0001 | 90.91 | 18.07 | Sig. improvement from BL |
| 2 | 4 | 5.03 | 5.03 | <0.0001 | 90.91 | 17.90 | Sig. improvement from BL |
| 2 | 6 | 5.07 | 5.07 | <0.0001 | 100.00 | 18.46 | Sig. improvement from BL |
| 2 | 8 | 2.62 | 2.67 | 0.0023 | 81.82 | 9.30 | Sig. improvement from BL |
| 3 | 2 | 0.21 | 0.07 | 0.9451 | 40.91 | 0.56 | No change from BL |
| 3 | 4 | 0.32 | 0.23 | 0.8116 | 59.09 | 0.87 | No change from BL |
| 3 | 6 | 0.21 | 0.07 | 0.9374 | 40.91 | 1.10 | No change from BL |
| 3 | 8 | -1.99 | -2.15 | 0.0132 | 22.73 | -7.35 | Sig. worsening from BL |
| 4 | 2 | -0.77 | -0.69 | 0.4733 | 59.09 | -1.44 | No change from BL |
| 4 | 4 | -0.06 | 0.07 | 0.9396 | 45.45 | 0.15 | No change from BL |
| 4 | 6 | -0.11 | 0.20 | 0.8155 | 59.09 | 0.88 | No change from BL |
| 4 | 8 | -3.33 | -3.00 | 0.0007 | 18.18 | -10.02 | Sig. worsening from BL |

As can be seen from Table 2, Sample 2 showed a statistically significant increase in hydration by the Corneometer at all timepoints. Samples 3 and 4 showed a statistically significant increase in hydration by the Corneometer at the 8-hour timepoint. "BL" in Table 5 refers to baseline. Baseline is the starting point for measurements.

Table 5: Skicon 200-EX: Skin surface hydration - CFB based on Log10 Transformed Values at the Volar Forearm

| Sample # | Time (4hr) | Mean CFB | Est. | p-value (two sided) | % subjects improvements from BL | Mean % improvement from BL | Conclusion |
|---|---|---|---|---|---|---|---|
| 2 | 2 | 0.19 | 0.20 | <0.0001 | 100 | 59.29 | Sig. improvement from BL |
| 2 | 4 | 0.23 | 0.23 | <0.0001 | 100 | 72.73 | Sig. improvement from BL |
| 2 | 6 | 0.22 | 0.22 | <0.0001 | 100 | 68.54 | Sig. improvement from BL |
| 2 | 8 | 0.21 | 0.21 | <0.0001 | 100 | 65.43 | Sig. improvement from BL |
| 3 | 2 | 0.03 | 0.03 | 0.0204 | 77.27 | 7.59 | Improvement from BL |
| 3 | 4 | 0.05 | 0.05 | 0.0005 | 86.36 | 13.04 | Improvement from BL |
| 3 | 6 | 0.07 | 0.07 | <0.0001 | 90.91 | 18.76 | Improvement from BL |
| 3 | 8 | 0.05 | 0.05 | 0.005 | 90.91 | 12.98 | Improvement from BL |
| 4 | 2 | 0.04 | 0.04 | 0.0049 | 81.82 | 9.78 | Improvement from BL |
| 4 | 4 | 0.06 | 0.06 | <0.0001 | 86.36 | 16.24 | Improvement from BL |
| 4 | 6 | 0.07 | 0.07 | <0.0001 | 86.36 | 17.20 | Improvement from BL |
| 4 | 8 | 0.06 | 0.06 | <0.0001 | 90.91 | 14.89 | Improvement from BL |

As seen in Table 5, Sample 2, the inventive sample showed a significant increase in hydration by Skicon at all time points and Samples 3 and 4 showed a slight increase in hydration at all time points.

Example 3

In this example, various combinations of benefit agents were tested for resilience wound healing and barrier repair. For wound healing assessments, cells were grown to 80% confluency and treated with bioactives for 24 to 36 hours. Post treatment, a scratch was created on the cell and closure of the wound was monitored after 24 hours of incubation. Closure of the scratch area was calculated as a % closure of the wound. Microscopic images were captured at a magnification of 4x and the open wound area measurements were analyzed using the software CellSens. Wound closure in percentage = (mean of the readings of the open wound area at (t=24) - mean of the readings of the open wound area at (t=0) / mean of the readings at (t=0)) *100. Table 6 lists the results from the wound healing assay.

Table 6

| Material | % Closure in Scratch Area |
|---|---|
| Control | 31 |
| Vitamin B3 0.2% | 30 |
| Vitamin E 0.001% | 32 |
| Vitamin E 0.05% | 48 |
| Aloe Vera 0.1% | 63 |
| Aloe Vera 0.01% | 55 |
| Sodium Hyaluronate 0.001% | 51 |
| Vitamin B3 0.2%, Vitamin E 0.001%, Aloe Vera 0.1% | 55 |
| Vitamin B3 0.2%, Vitamin E 0.001%, Aloe Vera 0.1%, Sodium Hyaluronate 0.001% | 60 |

As can be seen in Table 6, a significant improvement was observed in wound healing with the combination of Vitamin B3, Vitamin E, Aloe Vera, and Sodium Hyaluronate, a statistically significant improvement compared to Vitamin B3 or Vitamin E.

Example 4

In this example, a melanin content assay was performed to determine the inhibition or melanin reduction found with various combinations of benefit actives as seen in Table 7. In this Example, cells were grown for 24 hours and treated with bioactives and incubated for 72 hours. Post treatment, the cells were lysed and absorbance measured at 405 nm. The lysate was transferred to a fresh 384-well clear flat bottom plate and an original diameter was measured at 405 nm using a FlexStation 3 reader. (% reduction in melanin content was measured by taking the mean absorbance value of the control - the mean absorbance value of the treated cells / the mean absorbance value of the control) *100

Table 7

| Material | % Inhibition in Melanin Content |
|---|---|
| Vitamin B3 0.2% | 7.47 |
| Vitamin E 0.001% | 3.56 |
| Vitamin E 0.05% | 15.62 |
| Aloe Vera 0.01% | 19.45 |
| Aloe Vera 0.1% | 17.17 |
| Sodium Hyaluronate 0.001% | 9.09 |
| Vitamin B3 0.2%, Vitamin E 0.001%, Aloe Vera 0.1% | 17.19 |
| Vitamin B3 0.2%, Vitamin E 0.05%, Aloe Vera 0.1% | 23.57 |
| Vitamin B3 0.2%, Vitamin E 0.001%, Aloe Vera 0.1%, Sodium Hyaluronate 0.001% | 27.13 |

In this melanin content assay, 4-Hexyl resorcinol @ 10 micromolar gives ~33% inhibition (benchmark). It is generally considered in a melanin content assay that any inhibition above ~15% was be considered as significant. Thus, as can be seen from Table 7, the combination of Vitamin B3, Vitamin E, and Aloe Vera at both levels of Vitamin E showed significant inhibition in melanin content. Additionally, the combination of Vitamin B3, Vitamin E, Aloe Vera, and Sodium Hyaluronate showed significant inhibition in melanin content.

In the absence of explicitly stating otherwise, all ranges described herein are meant to include all ranges subsumed therein. As used herein, except where explicitly described, substantially free of means less than 10% by weight. Antimicrobial benefits mean at least a log kill of 2, preferably at least a log kill of 3, in under 3 minutes, or even 10 seconds whereby antimicrobial assessment is measured via ASTM International standard method E2783-11 (Reapproved 2016) which sets forth the procedure for measuring antimicrobial activity for water miscible compounds using a time kill procedure. Viral (or virus) inactivation is determined by assessing the impact of microbiocides against viruses as set forth in ASTM International standard method 1052-20. The term comprising is meant to encompass the terms consisting essentially of and consisting of. For the avoidance of doubt, and for illustration, a composition comprising water, cationic surfactant and preservative is meant to include a composition consisting essentially of the same and a composition consisting of the same.

Except where otherwise explicitly indicated, all numbers in this description indicating amounts of material or conditions of reaction, physical properties of materials and/or use are to be understood as modified by the word "about." All amounts are by weight of the final composition, unless otherwise specified.

It should be noted that in specifying any range of concentration or amount, any particular upper concentration can be associated with any particular lower concentration or amount as well as any subranges consumed therein. In that regard, it is noted that all ranges disclosed herein are inclusive of the endpoints, and the endpoints are independently combinable with each other (e.g., ranges of "up to 25% by weight, or, more specifically, 5% by weight to 20% by weight, in inclusive of the endpoints and all intermediate values of the ranges of 5% by weight to 25% by weight, etc.). "Combination is inclusive of blends, mixtures, alloys, reaction products, and the like. Furthermore, the terms "first", "second", and the like herein do not denote any order, quantity, or importance, but rather are used to distinguish one element from another. The terms "a" and "an" and "the" herein do not denote a limitation of quantity and are to be construed to cover both the singular and the plural, unless otherwise indicated herein or clearly contradicted by context. The suffix "(s)" as used herein is intended to include both the singular and the plural of the term it modifies, thereby including one or more of the term (e.g., the film(s) includes one or more films). Reference throughout the specification to "one embodiment", "one aspect", "another embodiment", "another aspect", "an embodiment", "an aspect" and so forth means that a particular element (e.g., feature, structure, and/or characteristic) described in connection with the embodiment or aspect is included in at least one embodiment or aspect described herein and may or may not be present in other embodiments or aspects. In addition, it is to be understood that the described elements may be combined in any suitable manner in the various embodiments or aspects.

While particular aspects have been described, alternatives, modifications, variations, improvements, and substantial equivalents that are or may be presently unforeseen may arise to applicants or others skilled in the art. Accordingly, the appended claims as filed and as they may be amended are intended to embrace all such alternatives, modifications, variations, improvements, and substantial equivalents.

For the avoidance of doubt the word "comprising" is intended to mean "including" but not necessarily "consisting of" or "composed of." In other words, the listed steps, options, or alternatives need not be exhaustive.

The disclosure of the invention as found herein is to be considered to cover all aspects as found in the claims as being multiply dependent upon each other irrespective of the fact that claims may be found without multiple dependency or redundancy. Unless otherwise specified, numerical ranges expressed in the format "from x to y" are understood to include x and y. In specifying any range of values or amounts, any particular upper value or amount can be associated with any particular lower value or amount. All percentages and ratios contained herein are calculated by weight unless otherwise indicated. The various features of the present invention referred to in individual sections above apply, as appropriate, to other sections *mutatis mutandis.* Consequently, features specified in one section may be combined with features specified in other sections as appropriate. Any section headings are added for convenience only and are not intended to limit the disclosure in any way.

## Claims

1. A transparent antibacterial composition, comprising:

water;
ethanol, propanol, isopropyl alcohol, or a combination thereof;
a humectant; and
a sensory oil; wherein the sensory oil must be liquid at room temperature, the sensory oil must be soluble in at least

EP 4 216 911 B1

75/25 EtOH/Water, and the sensory oil must have no solvency for water; and wherein the water is present in an amount of 8 to 30% by weight, preferably 10 to 25% by weight, more preferably 10 to 20% by weight and wherein the alcohol is present in an amount of 62 to 75% by weight, preferably 65 to 75% by weight.

2. The antibacterial composition of any of the preceding claims, wherein the sensory oil is present in an amount of 0.01 to 10% by weight, preferably 0.5 to 5% by weight.

3. The antibacterial composition of any of the preceding claims, wherein the sensory oil comprises dicarboxylic acid esters, lactate esters, triglycerides, soybean oil, fatty acid esters, (poly)propylene glycol fatty ethers or a combination thereof, wherein addition of the sensory oil does not affect the transparency of the antibacterial composition.

4. The antibacterial composition of Claim 3, wherein the sensory oil comprises a sensory oil which is soluble in at least 75/25 EtOH/Water, selected from diisopropyl adipate, diisopropyl sebacate, or diethyl hexyl malate, lauryl lactate, myristyl lactate, cetyl lactate, isostearyl alcohol, PPG15 stearyl ether or a combination thereof.

5. The antibacterial composition of any of the preceding claims, wherein the alcohol comprises ethanol, isopropanol, n-propanol, or a combination thereof.

6. The antibacterial composition of any of the preceding claims, wherein the humectant is present in an amount of 1 to 10% by weight, preferably 2 to 8% by weight, more preferably 3 to 7% by weight.

7. The antibacterial composition of any of the preceding claims, wherein the humectant comprises glycerin, butylene glycol, propylene glycol, polyethylene glycols, sorbitol, polyglycerol, isoprene glycol, hyaluronic acid, or a combination thereof.

8. The antibacterial composition of any of the preceding claims, further comprising a skin benefit agent.

9. The antibacterial composition of Claim 8, wherein the skin benefit agent comprises niacinamide, tocopherol, aloe vera, sodium or potassium hyaluronate, alpha-hydroxy acids and esters, beta-hydroxy acids and esters, hydroxyethyl urea, polyhydroxy acids and esters, creatine, hydroquinone, t-butyl hydroquinone, mulberry extract, licorice extract, or a combination thereof.

10. The antibacterial composition of any of the preceding claims, wherein the antibacterial composition is in the form a liquid, preferably wherein the antibacterial composition is in the form of a liquid spray antibacterial composition.

11. The antibacterial composition of any of the preceding claims, further comprising a chelating agent, preferably wherein the chelating agent comprises ethylene diaminetetraacetic acid (EDTA), diethylenetriaminepentaacetic acid (DTPA), ethylene diamine disuccinic acid (EDDS), pentasodium diethylenetriaminepentaacetate, trisodium N-(hydroxyethyl)-ethylenediaminetracetate, sodium thiocyanate, trisodium salt of methylglycinediacetic acid, tetrasodium glutamate diacetate and phytic acid, preferably wherein the chelating agent is ethylene diaminetetraacetic acid (EDTA), diethylenetriaminepentaacetic acid (DTPA), ethylene diamine disuccinic acid (EDDS), or a combination thereof.

12. A method of making a transparent antibacterial composition, comprising:

combining a sensory oil and a humectant forming a first phase;
combining water with ethanol, propanol, isopropyl alcohol, or a combination thereof forming a second phase;
combining the first phase and the second phase forming a third phase; and
adding a neutralizer to the third phase, thereby forming the antibacterial composition;
wherein the sensory oil must be liquid at room temperature, the sensory oil must be soluble in at least 75/25 EtOH/Water, and the sensory oil must have no solvency for water; and wherein the water is present in an amount of 8 to 30% by weight, preferably 10 to 25% by weight, more preferably 10 to 20% by weight and wherein the alcohol is present in an amount of 62 to 75% by weight, preferably 65 to 75% by weight.

13. The method of Claim 12, further comprising adding a fragrance to the antibacterial composition.

15

**Patentansprüche**

1. Transparente antibakterielle Zusammensetzung, umfassend:

   Wasser;
   Ethanol, Propanol, Isopropylalkohol oder eine Kombination davon;
   ein Feuchthaltemittel und
   ein sensorisches Öl;
   wobei das sensorische Öl bei Raumtemperatur flüssig sein muss, das sensorische Öl in mindestens 75/25 EtOH/Wasser löslich sein muss und das sensorische Öl kein Lösungsvermögen für Wasser haben darf und wobei das Wasser in einer Menge von 8 bis 30 Gew.-%, bevorzugt von 10 bis 25 Gew.-%, bevorzugter von 10 bis 20 Gew.-% vorliegt und wobei der Alkohol in einer Menge von 62 bis 75 Gewichts-%, bevorzugt von 65 bis 75 Gewichts-%, vorliegt.

2. Antibakterielle Zusammensetzung nach einem der vorhergehenden Ansprüche, wobei das sensorische Öl in einer Menge von 0,01 bis 10 Gewichts-%, bevorzugt von 0,5 bis 5 Gewichts-%, vorliegt.

3. Antibakterielle Zusammensetzung nach einem der vorhergehenden Ansprüche, wobei das sensorische Öl Dicarbonsäureester, Lactatester, Triglyceride, Sojaöl, Fettsäureester, (Poly)propylenglycolfettether oder eine Kombination davon umfasst, wobei die Zugabe des sensorischen Öls die Transparenz der antibakteriellen Zusammensetzung nicht beeinträchtigt.

4. Antibakterielle Zusammensetzung nach Anspruch 3, wobei das sensorische Öl ein sensorisches Öl umfasst, das in mindestens 75/25 EtOH/Wasser löslich ist, ausgewählt unter Diisopropyladipat, Diisopropylsebacat oder Diethylhexylmalat, Lauryllactat, Myristyllactat, Cetyllactat, Isostearylalkohol, PPG15-Stearylether oder einer Kombination davon.

5. Antibakterielle Zusammensetzung nach einem der vorhergehenden Ansprüche, wobei der Alkohol Ethanol, Isopropanol, n-Propanol oder eine Kombination davon umfasst.

6. Antibakterielle Zusammensetzung nach einem der vorhergehenden Ansprüche, wobei das Feuchthaltemittel in einer Menge von 1 bis 10 Gewichts-%, bevorzugt von 2 bis 8 Gewichts-%, bevorzugter von 3 bis 7 Gewichts-%, vorliegt.

7. Antibakterielle Zusammensetzung nach einem der vorhergehenden Ansprüche, wobei das Feuchthaltemittel Glycerin, Butylenglycol, Propylenglycol, Polyethylenglycole, Sorbitol, Polyglycerin, Isoprenglycol, Hyaluronsäure oder eine Kombination davon umfasst.

8. Antibakterielle Zusammensetzung nach einem der vorhergehenden Ansprüche, die außerdem ein Hautpflegemittel umfasst.

9. Antibakterielle Zusammensetzung nach Anspruch 8, wobei das Hautpflegemittel Niacinamid, Tocopherol, Aloe vera, Natrium- oder Kaliumhyaluronat, Alpha-Hydroxysäuren und -ester, Beta-Hydroxysäuren und -ester, Hydroxyethylharnstoff, Polyhydroxysäuren und -ester, Kreatin, Hydrochinon, t-Butylhydrochinon, Maulbeerextrakt, Lakritzextrakt oder eine Kombination davon umfasst.

10. Antibakterielle Zusammensetzung nach einem der vorhergehenden Ansprüche, wobei die antibakterielle Zusammensetzung in Form einer Flüssigkeit vorliegt, wobei die antibakterielle Zusammensetzung bevorzugt in Form einer flüssigen antibakteriellen Sprühzusammensetzung vorliegt.

11. Antibakterielle Zusammensetzung nach einem der vorhergehenden Ansprüche, die außerdem einen Chelatbildner umfasst, wobei der Chelatbildner bevorzugt Ethylendiamintetraessigsäure (EDTA), Diethylentriaminpentaessigsäure (DTPA), Ethylendiamindibernsteinsäure (EDDS), Pentanatriumdiethylentriaminpentaacetat, Trinatrium-N-(hydroxyethyl)-ethylendiamintetraacetat, Natriumthiocyanat, Trinatriumsalz von Methylglycindiessigsäure, Tetranatriumglutamatdiacetat und Phytinsäure umfasst, wobei der Chelatbildner bevorzugt Ethylendiamintetraessigsäure (EDTA), Diethylentriaminpentaessigsäure (DTPA), Ethylendiamindibernsteinsäure (EDDS) oder eine Kombination davon ist.

12. Verfahren zur Herstellung einer transparenten antibakteriellen Zusammensetzung, umfassend:

Kombinieren eines sensorischen Öls und eines Feuchthaltemittels zur Ausbildung einer ersten Phase;
Kombinieren von Wasser mit Ethanol, Propanol, Isopropylalkohol oder einer Kombination davon zur Ausbildung einer zweiten Phase;
Kombinieren der ersten Phase und der zweiten Phase zur Ausbildung einer dritten Phase, und
Zugabe eines Neutralisationsmittels zu der dritten Phase, wodurch die antibakterielle Zusammensetzung gebildet wird;
wobei das sensorische Öl bei Raumtemperatur flüssig sein muss, das sensorische Öl in mindestens 75/25 EtOH/Wasser löslich sein muss und das sensorische Öl kein Lösungsvermögen für Wasser haben darf und wobei das Wasser in einer Menge von 8 bis 30 Gew.-%, bevorzugt von 10 bis 25 Gew.-%, bevorzugter von 10 bis 20 Gew.-% vorliegt und wobei der Alkohol in einer Menge von 62 bis 75 Gewichts-%, bevorzugt von 65 bis 75 Gewichts-%, vorliegt.

13. Verfahren nach Anspruch 12, das außerdem das Hinzufügen eines Duftstoffs zu der antibakteriellen Zusammensetzung umfasst.

## Revendications

1. Composition antibactérienne transparente, comprenant:

de l'eau;
de l'éthanol, du propanol, de l'alcool isopropylique ou une combinaison de ceux-ci;
un humectant; et
une huile sensorielle; où l'huile sensorielle doit être liquide à température ambiante, l'huile sensorielle doit être soluble dans EtOH/eau à au moins 75/25, et l'huile sensorielle ne doit pas avoir de pouvoir solvant pour l'eau; et où l'eau est présente en une quantité de 8 à 30 % en poids, de préférence de 10 à 25 % en poids, de préférence encore de 10 à 20 % en poids et où l'alcool est présent en une quantité de 62 à 75 % en poids, de préférence de 65 à 75 % en poids.

2. Composition antibactérienne selon l'une quelconque des revendications précédentes, où l'huile sensorielle est présente en une quantité de 0,01 à 10 % en poids, de préférence de 0,5 à 5 % en poids.

3. Composition antibactérienne selon l'une quelconque des revendications précédentes, où l'huile sensorielle comprend des esters d'acides dicarboxyliques, des esters lactates, des triglycérides, de l'huile de soja, des esters d'acides gras, des éthers gras de (poly)propylèneglycol ou une combinaison de ceux-ci, où l'addition de l'huile sensorielle n'affecte pas la transparence de la composition antibactérienne.

4. Composition antibactérienne selon la revendication 3, où l'huile sensorielle comprend une huile sensorielle qui est soluble dans EtOH/eau à au moins 75/25, choisie parmi l'adipate de diisopropyle, le sébaçate de diisopropyle ou le malate de diéthylhexyle, le lactate de lauryle, le lactate de myristyle, le lactate de cétyle, l'alcool isostéarylique, l'éther stéarylique de PPG15 ou une combinaison de ceux-ci.

5. Composition antibactérienne selon l'une quelconque des revendications précédentes, où l'alcool comprend de l'éthanol, de l'isopropanol, du n-propanol ou une combinaison de ceux-ci.

6. Composition antibactérienne selon l'une quelconque des revendications précédentes, où l'humectant est présent en une quantité de 1 à 10 % en poids, de préférence de 2 à 8 % en poids, de préférence encore de 3 à 7 % en poids.

7. Composition antibactérienne selon l'une quelconque des revendications précédentes, où l'humectant comprend de la glycérine, du butylèneglycol, du propylèneglycol, des polyéthylèneglycols, du sorbitol, du polyglycérol, de l'isoprèneglycol, de l'acide hyaluronique ou une combinaison de ceux-ci.

8. Composition antibactérienne selon l'une quelconque des revendications précédentes, comprenant en outre un agent bénéfique pour la peau.

9. Composition antibactérienne selon la revendication 8, où l'agent bénéfique pour la peau comprend du niacinamide, du tocophérol, de l'aloe vera, du hyaluronate de sodium ou de potassium, des alpha-hydroxyacides et esters, des bêta-hydroxyacides et esters, de l'hydroxyéthylurée, des polyhydroxyacides et esters, de la créatine, de l'hydroquinone, de la t-butylhydroquinone, de l'extrait de mûrier, de l'extrait de réglisse ou une combinaison de ceux-ci.

10. Composition antibactérienne selon l'une quelconque des revendications précédentes, où la composition antibactérienne est sous la forme d'un liquide, de préférence où la composition antibactérienne est sous la forme d'une composition antibactérienne à pulvériser liquide.

11. Composition antibactérienne selon l'une quelconque des revendications précédentes, comprenant en outre un agent chélatant, de préférence où l'agent chélatant comprend de l'acide éthylènediaminetétraacétique (EDTA), de l'acide diéthylènetriaminepentaacétique (DTPA), de l'acide éthylènediaminedisuccinique (EDDS), du diéthylènetriaminepentaacétate pentasodique, du N-(hydroxyéthyl)-éthylènediaminetraacétate trisodique, du thiocyanate de sodium, du sel trisodique d'acide méthylglycinediacétique, du diacétate de glutamate tétrasodique et de l'acide phytique, de préférence où l'agent chélatant est l'acide éthylènediaminetétraacétique (EDTA), l'acide diéthylènetriaminepentaacétique (DTPA), l'acide éthylènediaminedisuccinique (EDDS), ou une combinaison de ceux-ci.

12. Procédé de production d'une composition antibactérienne transparente, comprenant:

la combinaison d'une huile sensorielle et d'un humectant formant une première phase;
la combinaison d'eau avec de l'éthanol, du propanol, de l'alcool isopropylique ou une combinaison de ceux-ci formant une deuxième phase;
la combinaison de la première phase et de la deuxième phase formant une troisième phase; et
l'addition d'un neutralisant à la troisième phase, formant ainsi la composition antibactérienne;
où l'huile sensorielle doit être liquide à température ambiante, l'huile sensorielle doit être soluble dans EtOH/eau à au moins 75/25, et l'huile sensorielle ne doit pas avoir de pouvoir solvant pour l'eau, et où l'eau est présente en une quantité de 8 à 30 % en poids, de préférence de 10 à 25 % en poids, de préférence encore de 10 à 20 % en poids et où l'alcool est présent en une quantité de 62 à 75 % en poids, de préférence de 65 à 75 % en poids.

13. Procédé selon la revendication 12, comprenant en outre l'addition d'un parfum à la composition antibactérienne.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016058902 A1
- US 20160000669 A1

- US 6294182 B1

**Non-patent literature cited in the description**

- **KALIA et al.** Nanofibrillated cellulose: surface modification and potential applications. *Colloidal Polymer Science*, 2014, vol. 292, 5-31